# EUROPEAN PATENT APPLICATION

(11) **EP 2 133 425 A1**
(43) Date of publication of application: **16.12.2009**
(21) Application number: 09075260.1
(22) Date of filing: 09.06.2009
(51) Int. Cl.: C12N 15/11

(54) **Method and compound for antiviral (HIV) therapy**

(30) Priority: 13.06.2008 EP 08075554
(71) Applicant: Mölling, Karin, 14195 Berlin Stahnsdorf (DE)
(72) Inventor: Mölling, Karin, 14195 Berlin Stahnsdorf (DE)
(74) Representative: Boeckh, Tobias

(57) **Abstract**

The invention refers to an anti-viral therapeutic that inactivates the Human Immunodeficiency Virus (HIV) RNA by siDNA. The siDNA antiviral therapeutic is effective in the treatment and inactivation of cell free virus particles before infection and / or in the treatment and prevention of HIV infections inside the cell. The invention exploits the HIV RNase H activity of the reverse transcriptase which is essential for viral replication, causing premature cleavage and degradation of the viral RNA genome.

## Description

The invention refers to an anti-viral therapeutic that inactivates the Human Immunodeficiency Virus (HIV) RNA by siDNA. The siDNA antiviral therapeutic is effective in the treatment and inactivation of cell free virus particles before infection and / or in the treatment and prevention of HIV infections inside the cell. The invention exploits the HIV RNase H activity of the reverse transcriptase which is essential for viral replication, causing premature cleavage and degradation of the viral RNA genome. This is achieved by partially double-stranded, hairpin-loop-structured oligodeoxynucleotides. These oligonucleotides comprise one strand which is fully complementary to the 3'-polypurine tract PPT of HIV RNA and creates a local DNA/RNA hybrid as substrate for the virion-associated RNase H. The oligonucleotide contains a second strand partially complementary to the antisense strand, which protects against nucleases. Both strands are connected by a linker of four thymidines. The presence of G-clusters can assist higher order structure formation within or between siDNA molecules and enhances the anti-viral function. Triple-helix formation between siDNA and the target RNA is a preferred effect. The siDNA is superior to siRNA because the formation of RNA-DNA hybrids is preferred over double-stranded DNA or double-stranded RNA, which forms as a tertiary structure in RNA genomes, siDNA is easier to synthesize and is more stable than siRNA, and siDNA leads to HIV particle inactivation outside the cell before infection, whereas siRNA has no such activity. It can be combined with other siDNAs targeted against various virus strains in a cocktail; it can also be combined with siRNA to target early and late steps in viral replication.

### BACKGROUND OF THE INVENTION

Human Immunodeficiency Virus (HIV) is a lentivirus of the retrovirus family, and can lead to acquired immunodeficiency syndrome (AIDS), a condition in which the immune system fails leading to susceptibility to a wide range of diseases. HIV infection is pandemic in humans and until the present time no cure or vaccine has been developed. Current anti-retroviral drugs are expensive and often ineffective in counteracting the disease, thus there is an enormous need for the development of new antiviral therapeutics for the treatment and/or prevention of HIV infection.

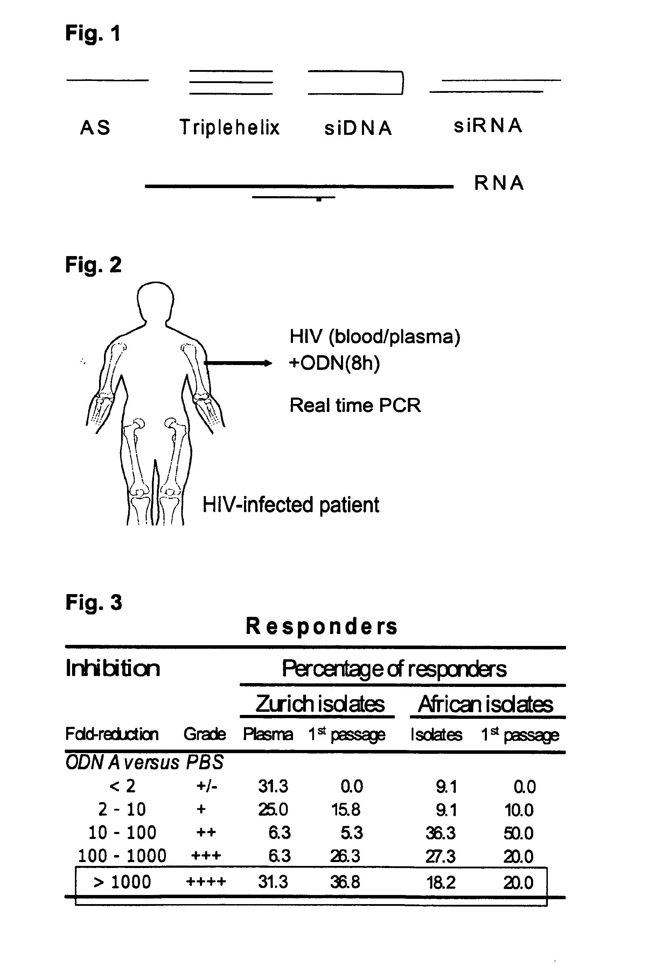

There are several ways to inactivate viral RNA genomes or RNAs such as messenger mRNAs inside the cell. One of these is the well-known antisense strategy, whereby a single stranded DNA oligonucleotide is directed to a target RNA and hybridizes by Watson-Crick based pairing. The RNA-DNA hybrid is recognized by an enzyme designated as ribonuclease H or RNA H, whereby H indicates the RNA-DNA hybrid substrates. It cleaves the RNA within the hybrid region (illustrated in Fig. 1).

A second mechanism is based on a triple helix, whereby the target strand may be single or double stranded DNA and the third strand binds to this double stranded structure. Again one strand hybridizes to the target strand by Watson-Crick base pairing, whereas the third strand binds to Hoogsteen base-pairing. A triple helix is characterized by its resistance to cleavage by cellular enzymes and leads to inhibition of expression by unknown mechanisms, possibly translation arrest. The role of a triple helix inside the cell is unclear (illustrated in Fig. 1).

A third mechanism is based on inactivating a target RNA by double stranded RNA, the so-called 'short interfering' siRNA. Here one strand of the siRNA is polyhybridized to the target RNA by Watson Crick base pairing, the so-called antisense strand. A second RNA strand, the passenger strand, is fully complementary to the antisense strand and only transiently required for inactivation of the RNA. It is removed in a so-called RISC-complex. This mechanism of inactivation of the target RNA by siRNA is called silencing. The enzyme required for RNA silencing is designated as Argonaute. The discovery of this mechanism was awarded with the Nobel Prize in 2006. In all cases gene inactivation is achieved by small oligonucleotides about 20 nucleotides in length, which may or may not contain a linker region between the two nucleic acid strands (illustrated in Fig. 1).

A fourth mechanism is the use of short interfering DNA (siDNA) to inactivate viral RNA genomes (illustrated in Fig.1). As shown by the inventor, this mechanism activates the RNase H of the virus and leads to silencing of the viral RNA (Matzen et al, Nature Biotechnol. 25, 669-674 (2007)). The RNase H is a Hybrid-specific RNase which was discovered by Moelling et al (Nature New Biology 234, 240-243 (1971)). The inventor also observed that cellular RNase H-like activities such as RNase HI and RNase H2A, B, C and even Agonaute 2 contribute to siDNA-mediated silencing. Agonaute 2 (Ag02) is the enzyme known to induce siRNA-mediated silencing. The inventor showed that Ag02 is enzymatically related to RNases H and can induce siDNA-mediated silencing as well (Moelling et al. Cold Spring Harb. Symp. Quant. Biol. 71, 365-368 (2006), Small Regulatory RNAs).

Furthermore, it could be shown by the inventor that siDNA is able to induce silencing of an oncogenic retrovirus, the Spleen Focus-Forming Virus and prevent infection, cause delay of disease progression and lead to increased survival time (Matzen et al. Nature Biotechnol. 25, 669-674 (2007)). Furthermore the inventor showed in several cases that siDNA is superior to a single-stranded antisense effect (Jendis et al, AIDS Research and Human Retroviruses 12, 1161-1168 (1996), AIDS Research and Human Retro-viruses 14, 999-1005, (1998), Moelling et al, FEBSLetters, 580, 3545-3550 (2006), Matzen et al. Nature Biotechnol. 25, 669-674 (2007)).Thus the effect of siDNA has been demonstrated in several cases.

The four mechanisms described are all distinct from each other.

### SUMMARY OF THE INVENTION

One problem to be solved with the present invention is to present an effective antiviral therapeutic against cell-free HIV particles and HIV infections using siDNA. This problem may be solved by the features of the independent claims.

An object of the invention is therefore to present siDNA oligonucleotides, capable of binding to one or more RNA target regions of HIV, as an antiviral therapeutic for the treatment and inactivation of cell free virus particles before infection and / or for the treatment and prevention of HIV infections inside the cell, whereby the siDNA oligonucleotides comprise or consist of an antisense-strand homologous to the viral RNA target and a second strand, partially complementary to the antisense-strand.

Another object of the invention is therefore to present siDNA oligonucleotides, capable of binding to one or more RNA target regions of HIV, as an antiviral therapeutic for the treatment and inactivation of cell free virus particles before infection and / or for the treatment and prevention of HIV infections inside the cell, whereby the siDNA oligonucleotides are comprising or consisting of an antisense-strand fully or almost fully homologous to the viral RNA target and a second strand, partially complementary to the antisense-strand forming a partially double stranded hairpin-loop-structured oligonucleotide, comprising G-clusters consisting of or including at least two G nucleotides in succession to allow tetrade or tetramer formation or tetra-helices or higher-ordered structures within the same siDNA oligonucleotide molecule (cis conformation) or through interaction with another siDNA oligonucleotide molecule (*trans* conformation).

Another object of the invention is to provide a method, respectively the use of siDNA oligonucleotides, or a combination of two or three siDNAs, as an antiviral therapeutic for the treatment and inactivation of cell free virus particles before infection and / or for the treatment and prevention of HIV infections inside the cell, capable of binding to RNA target regions of HIV or different HIV variants, whereby different siDNA oligonucleotides may be combined in a cocktail as pharmaceutical agent.

A further object of the invention is to provide a pharmaceutical composition as an antiviral therapeutic for the treatment and inactivation of cell free virus particles before infection and / or for the treatment and prevention of HIV infections inside the cell, comprising said siDNA oligonucleotides capable of binding to RNA target regions of HIV as an antiviral therapeutic.

The present invention describes a mechanism for the inactivation of an RNA target, preferentially of viral origin, such as retroviruses or retrovirus-like viruses or elements, using a short partially double stranded DNA. This so-called short DNA (sDNA) forms a hairpin loop structure. One arm serves as antisense arm targeted to the RNA of interest. A local RNA-DNA hybrid is formed, which is a substrate for RNase H. In the case of retroviruses, such as HIV, the RNase H is of viral origin, coded for the viral genome and is even present is cell-free virus particles. It is required inside the cell immediately after infection and therefore carried into the cell by the virus particle. As in the above-mentioned cases, the antisense arm is about 20 to 30 nucleotides long and the second DNA strand has been selected by Hoogsteen base pairing rules. The retroviral RNase H is pre-maturely activated by the siDNA for self-destruction of the HIV RNA genome. The viral RNA is inactivated at the site
where the local RNA-DNA hybrid forms due to cleavage by the viral RNase H.

The RT/RNase H is located inside the virus particles and is carried into the cell during infection. During RT- mediated reverse transcription RNA-DNA hybrids are generated. The RNase H removes the RNA in the hybrids, but leaves the polypurine tract (PPT) RNA intact. The PPT is required as primer for initiation of second strand DNA synthesis. An oligodeoxynucleotide (ODN) that specifically binds the PPT of HIV-1 forms a local RNA-DNA hybrid that mimics a natural replication intermediate. This hybrid activates RNA hydrolysis by the HIV RT/RNase H and destroys the viral RNA template before DNA synthesis. A partially double-stranded oligo inhibits HIV-1 replication in infected cells, including drug-resistant strains of HIV. Furthermore, because the RT/RNase H is associated with virus particles, they can be inactivated outside of the cell and this renders the virus non-infectious. The effect of such oligos is sequence-specific and a partially double-stranded ODN is more inhibitory than a single-stranded antisense ODN. G-tetrades also appear to be important in the function of the oligos.

Therefore, siDNA oligonucleotides, capable of binding to one or more RNA target regions of HIV are claimed as antiviral therapeutics, whereby the siDNA oligonucleotides are consisting of an antisense-strand homologous to the viral RNA target and a second strand, partially complementary to the antisense-strand forming a hairpin-loop structure. The siDNA oligonucleotides correspond to one or more HIV target regions of at least 20 nucleotides in length, whereby the antisense strand of siDNA is fully or almost fully (more than 80%, more preferably more than 90%) homologous to the target HIV viral RNA. Further, the second strand of the siDNA oligonucleotides is connected to the antisense strand through a thymidine linker, preferred - but not exclusively - 4 nucleotides in length, and whereby further the second strand is partially (40 - 60% homology) complementary to the antisense-strand and may be able to form triple helices by non-Watson-Crick base pairing with the viral RNA target strand.

Conserved target RNA regions are preferred due to the lower variation of DNA sequence between strains or variants, thus creating a higher likelihood of siDNA-RNA target pairing. Targeting a subunit of the transcriptase, and thus its silencing, would also reduce viral RNA replication efficiently. Furthermore, siDNA can also be targeted to cellular mRNAs coding for proteins essential for virus replication and indirectly prevent HIV replication.

Also a preferred embodiment of the invention is the use of a combination of two or three siDNAs as described herein as an antiviral therapeutic capable of binding to RNA target regions of HIV. HIV has a high genetic variability due to its fast replication cycle, high mutation rate and the potential generation of recombinant strains. Because of this a further preferred embodiment is the use of a cocktail of different siDNA oligonucleotides in a pharmaceutical agent to target different HIV variants that have or may have infected the patient.

The siDNA can be applied to an infected cell or an infected individual with a transducing agent, whereby the transducing agent is selected from the following group: the virus itself, a replicating HIV particle, which carries the siDNA into the cell during the process of infection, a liposome, transmembrane carriers, peptides. Most preferred is a cell transfection by using a lipid-based transfection reagent like Lullaby which is known from siRNA silencing. Other lipid based transducing agents are preferred, too.

It is an unexpected unique embodiment of the invention that siDNA acts early on the virus replication cycle and holds promise for the prevention of infection. The mechanism employed by this invention is a novel anti-viral approach in that virus particles can be inactivated outside the host cell, rendering HIV susceptible to treatment before infection of the host immune system.

A pharmaceutical composition prepared according to the invention is especially intended for the prevention of viral infection (by inactivation of the virus particle itself (outside the cell, before infection)), especially during sexual intercourse. The invention is further important to prevent mother to child transmission during birth delivery by applying the pharmaceutical composition to the mother shortly before birth delivery. Further, the invention is of importance for any kind of surgery (also oral and dental surgery) in respect of prevention virus transmission during surgery by reduction of virus load in body fluids (i.e. blood, saliva). Another mode of application could be Pre-Exposure Prophylaxis (PrEP) - a treatment that would counteract a major route of infection in the third world.

One significant advantage of the invention over siRNA treatment is that inactivation of virus particles outside the cell cannot be accomplished by siRNA. The treatment of RNA viruses by siRNA is dependent on cell infection. No other HIV antiviral treatment known at this time shows destructive activity against cell free HIV particles.

This invention provides suprising and unexpected success in the inactivation of HIV both *in vitro* and *in vivo* via a novel anti-viral mechanism, as demonstrated in the examples below. As demonstrated in vitro, the invention leads to a significant reduction of plasma HIV RNA levels and a subsequent reduction in the infectivity of treated virions. As demonstrated in vivo, infection of a lentiviral HIV-analogue and HIV infection of a humanised immune deficient (huPBL/SCID) mouse model are reduced after treatment with the claimed antiviral therapeutic. Due to induced self-destruction of HIV - observed with clinical samples - premature activation rather than inhibition of a viral enzyme is an effective tool for antiretroviral control, and provides significant advantages over existing inhibition-based HIV therapeutics.

### DETAILED DESCRIPTION AND PREFFERED EMBODIMENTS OF THE INVENTION

Under the term "homology" the following should be understood: An siDNA oligonucleotide according to the invention is consisting of an antisense-strand which is more than 80% homologous to the viral RNA target and a second strand, which is partially complementary to the antisense-strand forming a partially double stranded hairpin-loop-structure. The term "partially complementary" means a homology between 40 and 60% within the hairpin-loop.

Under the term "G-cluster" the following should be understood: "G-clusters" according to the invention are motifs within a sequence of an siDNA oligonucleotide. A G-cluster is defined by at least 2 G nucleotides in succession ("GG"). Several G-clusters lead to tetrade or tetramer formation or tetra-helices or higher-ordered structures within the siDNA oligonucleotide molecule. Each G-cluster is separated from another G-Cluster by 1 to 20 X nucleotides, wherein X is A, T, C or a single G, followed by A, T or C. General examples (among others) according to said definition are
i. GG
ii. GGG
iii. GGXG
iv. GGGG
v. GGXXXGGXXXXGG
vi. GGXXGGXXXXGGG

G-tetrade formation may be beneficial consisting e.g. of GGXXGGXXXXGGG etc, whereby X represents basically other nucleotides than "GG", such as A, T or C or a single G, as long as the single G is followed by A, T or C. The siDNA oligonucleotides according to the invention contain G clusters to allow tetrade or tetramer formation or tetra-helices by those sequences. The hairpin-loop structure may consist preferably of up to 9 non-self-complementary sequences at the 3'- and 5'-ends, followed by 6base-pairing, 2non-pairing, 3pairing, 2non-pairing, 3pairing sequences, followed by 4Ts. A homology of 40 to 60 % is preferred.

The tetrade or tetramer formation or tetra-helices or higher-ordered structures within the siDNA oligonucleotide molecule results of an interaction of G-clusters within the same siDNA oligonucleotide molecule (so-called cis conformation) or through interaction with another siDNA oligonucleotide molecule (so-called trans conformation). Both conformations (cis or trans) are possible.

Preferred specific examples according to the invention are siDNA oligonucleotides comprising the sequences SEQ ID NO 1 or SEQ ID NO 21 (framed sequence motifs are able to form tetrads, tetramers or tetra-helices or higher-ordered structures; bold nucleotides indicate G-cluster): 
ODN A (= SEQ ID NO 1):

5'-TTTTCTTTTGGGGGGTTTGGTTGGGTTTTCCCTTCCAGTCCCCCCTTTTCTTTT-3'
 
ODN AM (= SEQ ID NO 21):

5′-
mUmUmUmUCTTTTGGGGGGTTTGGTTGGGTTTTCCCTTCCAGTCCCCCCTTTTCmUmU-
mUmU-3′
 ["mU" indicates 2'-O-methylated uridine]

The invention is based on the cognition that a DNA strand has a thermodynamic preference to form an RNA-DNA hybrid over double-stranded DNA or double-stranded RNA. Thus the invention is based on the unexpected result that siDNA is of high preference (in contrast to siRNA) to form RNA-DNA hybrids; siDNA is therefore of advantage and a preferred object of the invention. Furthermore siDNA acts against the newly infecting incoming viral RNA, 2 to 3 days before siRNA. Thus a virus infection can be prevented by siDNA only. In contrast thereto, interferon-induction is a risk for siRNA and reduced or absent for an RNA-DNA hybrid or double-stranded DNA. Furthermore, siDNA is preferred due to the fact that DNA is easier to synthesize and more stable.

Also, an siDNA-containing pharmaceutical agent can also contain ribonucleotides, siDNA/RNA chimeras (as ODN AM, SEQ ID NO 21), or can be combined with siRNAs. However, the RNase H cleavage site needs to consist of a local RNA-DNA hybrid.

siDNA is also superior to a simple antisense Oligodeoxynucleotide, because it is more stable during uptake and inside the cell, and is therefore more effective. It acts earlier than antisense DNA, and can target incoming RNA, while antisense DNA targets mRNA. siDNA targets viral RNA before replication while siRNA is effective only late during replication targeting the mRNA.

Furthermore, siDNA oligonucleotides can be stabilized by base modifications. Such base modifications entail 2'-O-methylated nucleotides and phosphorothioates at the ends and in the central linker regions to protect against nucleases and to increase stability and longevity both in vivo and in pharmaceutical preparations. Other chemical modifications are also envisaged that may improve the stability of the siDNA oligonucleotides.

### Materials and Methods used in the Following Examples

### Patients

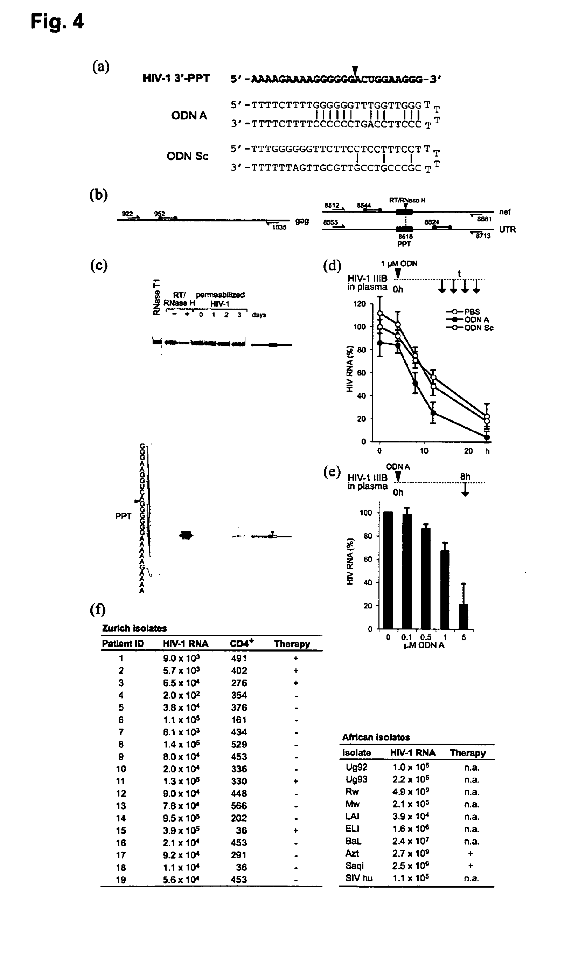

We obtained HIV-infected blood from 20 patients from the Zurich University hospital with a broad range of history, HAART, ART or no therapies, high or low viral loads and CD4 counts (see Fig. 4F). Furthermore we obtained HIV-1 field isolates from Africa Uganda-92 (Ug92), Uganda-93 (Ug93), Rwanda (Rw), Malawi (Mw), HIV-1 ELI, HIV-1 LAI/BRU, HIV-1 BaL, HIV-1 IIIB as well as strains resistant to the nucleoside analogue AZT (Azt) and the protease Inhibitor Saquinavir (Saqi) from the National Institute of Health (NIH) AIDS Research and Reference Reagent Program.

### Plasma, PBMCs and HIV

Plasma was extracted from 10 ml of blood of HIV-1 infected patients (available through the Department of Infectious Diseases of the University Hospital, University of Zurich, Switzerland) using the BD Vacutainer CPT method (Becton Dickinson, USA). Overview of the patients' isolates from Zurich is presented in Fig. 4F.

Primary Peripheral Blood Mononuclear Cells (pPBMCs) and plasma were extracted from fresh blood obtained from healthy donors (available through the Blood Donation Center, Zurich, Switzerland) using Ficoll Paque Plus methods (GE Health Technologies, USA). Sucrose-purified virions from patient 8898 used for cleavage assays were a kind gift from Dr. J. Boeni from the Swiss National Center for Retroviruses, Zurich, Switzerland.

### Oligodeoxynucleotides

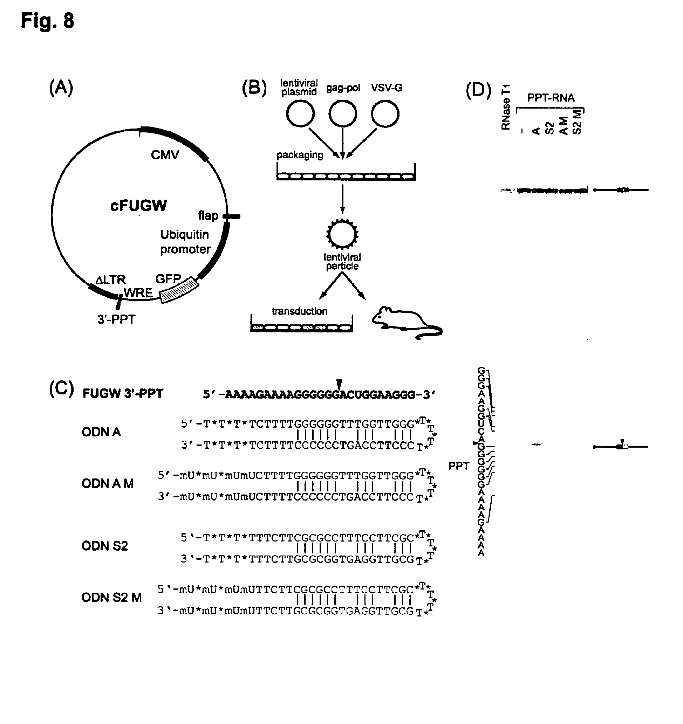

The ODN A (SEQ ID NO 1) consists of a 25mer antisense strand of PPT, and a 25mer passenger strand, connected by four thymidines (Fig. 4a). ODN Sc, which has the same length and nucleotide composition as ODN A but a randomized sequence of both strands served as a control for non-specific activity of ODN A (Fig. 4a). The ODNs were phosphorothioated at each end (3 bases) and in the T4 linker.
ODN AM (SEQ ID NO 21) is the methylated version of the ODN A (Fig. 8C). ODN S2 consists of a randomized sequence of both strands serving as a control for non-specific activity of ODN A. ODN S2M is therefore the methylated sequence of the ODN S2 (Fig. 8C).
The ODNs were purchased from Operon (Germany) or Integrated DNA Technology (USA).

### RNA isolation

Viral RNA was isolated from plasma or cell supernatants using the QIAamp Viral RNA Mini Kit (Qiagen, Germany) according to the manufacturer's directions.

### Determination of HIV RNA by qRT-PCR

HIV-1 RNA levels were determined by quantitative reverse transcription polymerase chain reaction (qRT-PCR) using RNA extracted from plasma or cell-culture supernatants as indicated in the legends. Isolated viral RNA was reverse transcribed using High-Capacity cDNA Archive Kit (Applied Biosystems, USA) and qRT-PCR was then performed using the Taqman Universal PCR master mix and the ABI 7300 instrument from Applied Biosystems. Location of the PCR primers and probes on the HIV-1 genome are schematically indicated in Fig. 4b. Coordinates for the primers and probes refer to HIV Gene Bank accession number 9629357. Sequences were: gag forward primer, 5'-GCAGCCATGCAAATGTTAAAAGAG-3' (SEQ ID NO 2); gag reverse primer, TCCCCTTGGTTCTCTCATCTGG (SEQ ID NO 3); FAM-TAMRA-gag probe, TCTATCCCATTCTGCAGCTTCCTCATT (SEQ ID NO 4); UTR forward primer, CAATGACTTACAAGGCAGCTGTAGA (SEQ ID NO 5); UTR reverse primer, TTAGCAGAACTACACACCAGGGC (SEQ ID NO 6); FAM-TAMRA-UTR probe, TTCACTCCCAAAGAAGACAAGATATCCTTGAT (SEQ ID NO 7). For detection of viral RNA of patients 6 and 9 Sybr-Green was used. All primers and the gag probe were purchased from Microsynth (Switzerland), the nef and UTR probes from Applied Biosystems. The results are presented in the figures as HIV RNA corresponding to absolute copy numbers per assay unless otherwise stated.

### Determination of HIV RNA in virions

HIV-1 virions, derived from 20 µl of patient plasma were incubated with different concentrations of ODNs. At indicated time points viral RNA was purified and the amount of undigested RNA was quantified by qRT-PCR using sets of primers covering the PPT region of the HIV genome, nef or UTR. Samples (3 µl) obtained from ARRRP were diluted to 20 µl in RPMI containing 20% FBS and treated similarly. qRT-PCR was performed using nef-primers.

### Test for infectivity of cell-free HIV on PBMCs

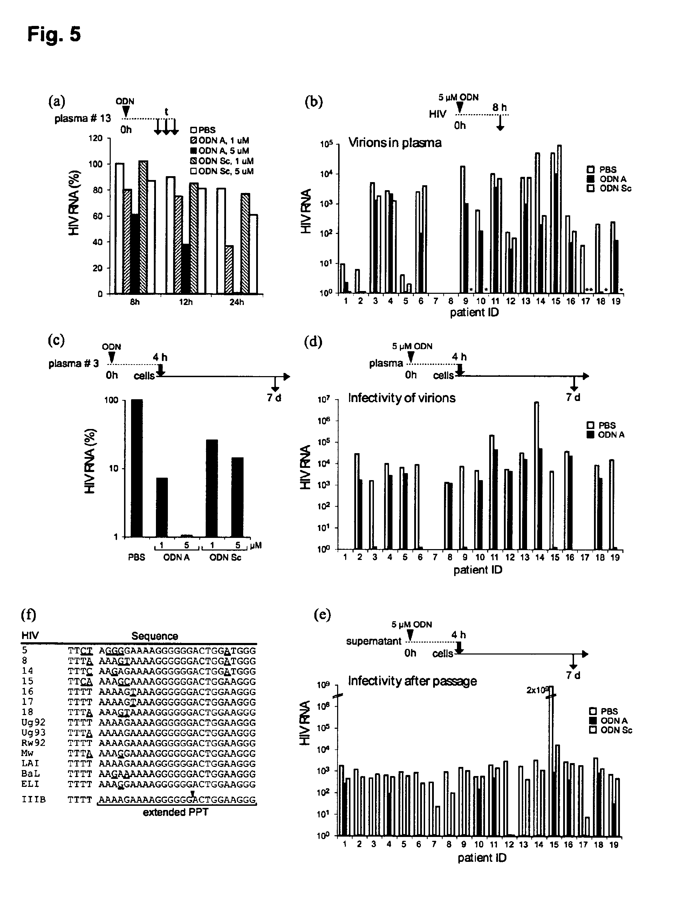

100µl of HIV-containing plasma from patients (Zurich isolates) or 5 µl of the African isolates were treated with either 1 or 5 µM of ODNs for 1 or 4 hours. 1 x 10⁶ Phytohemagglutinin (PHA)-activated PBMCs were infected with pre-treated HIV-1 virions and grown in culture at 37°C for 3 days in a total volume of 5ml of RPMI-1640 with 20 % FBS in the presence of interleukin 2 (IL-2, 20 U/ml, Zeptometrix, USA). At day 3, 3ml of RPMI-1640, 20 % FBS and IL-2 were added. At day 7 HIV RNA levels were analyzed in the supernatants using gag-specific qRT-PCR (Fig. 4B). For the experiment in Fig. 5E the equivalents of approximately 2 x10⁴ HIV RNA copies were used.

### DNA Sequencing

RNA isolated from HIV-1 infected patients and cell culture supernatants was reverse transcribed using random primers and High-Capacity cDNA Archive Kit (Applied Biosystems, USA) and the cDNA was sequenced in the HIV-1 PPT-flanking genomic regions using forward primer CCTCAGGTACCTTTAAGACCAATGAC (SEQ ID NO 8) and reverse primer CCCCTGGCCCTGGTGTGTA (SEQ ID NO 9). Thermal cycle sequencing was performed on a thermocycler (Touchgene from Genius, USA) for 25 cycles with either the forward or reverse primer using 10 ng of DNA templates. Purified sequencing products were run on an automated ABI 3100 genetic analyzer (Applied Biosystems). Sequences of both strands were analyzed and edited individually using the software package Sequencer 4.7 (Gene-Codes, USA), assembled as contigs with Sequence Assembler (Applied Biosystems) and subsequently aligned through Clustal-W algorithm of MacVector 7.1 software program (Accerlerys, USA).

### RT/RNase H cleavage assay

HIV-PPT RNA was in vitro transcribed, dephosphorylated and 5'-phosphorylated. Different ODNs (10 nM) and HIV-PPT RNA (10 nM) in RT buffer were annealed (2 min 90°C, 10 min 37°C) and cleaved with 0.1 U/µl RT/RNase H (GE Healthcare, USA) for 30 min at 37°C. The samples were subjected to electrophoresis in 10% polyacrylamide containing 8 M urea, together with HIV-PPT RNA partially digested with RNase T1 (Ambion) as described by the manufacturer.

### Lentiviral vector Production

FUGW is a lentiviral self-inactivating vector with a VSV-G coat, which allows transduction of many cell types but does not replicate. This lentiviral vector contains the PPT sequence that is identical to that of HIV. For generating high titer lentiviral vectors, 7x10⁶ human embryonal kidney (HEK) 293T cells in 10 cm plates were cotransfected with cFUGW, pCMVΔR8.9, and pHCMV-G (5 µg each) using lipofectamine 2000 (Invitrogen). Six hours post-transfection 8 ml fresh medium replaced the original medium. Two days post-transfection the culture media was collected and filtered through a 0.45 µm filter. The filtrated medium was then ultracentrifuged twice for 90 min using Polyallomer tubes. Titers of viral preparations were assessed by infecting 5x10⁵ C81-66 cells with serial dilution of the virus and determining the fraction of GFP-positive cells one day after infection by FACS. Titers of 5-9x10⁷ transducing units (TU) per ml were routinely obtained.

### Virions studies

FUGW virions (10⁴ TU) were either treated with different concentrations of ODN A (0-25 µM) for titration or different ODNs for 4 h at 37°C. These virions were also titrated using either 10⁴ IU or 10⁵ IU with 5 µM ODN A for also 4h at 37°C. Viral RNA was then extracted using the viral RNA mini kit (Qiagen) and eluted with 50 µl elution buffer. Then 5 µl RNA was used for cDNA synthesis in a reaction volume of 12.5 µl and 5 µl of the cDNA was used for qPCR.

### In vitro studies

Both cell lines VK2/E6E7 and Ect1/E6E7 were purchased from American Type Culture Collection (ATCC). The VK2/E6E7 (ATCC CRL-2616) cell line was established from normal vaginal mucosal tissue taken from a premenopausal woman undergoing anterior-posterior vaginal repair surgery. The ectocervical Ect1/E6E7 (ATCC CRL-2614) cell line was established from normal epithelial tissue taken from a premenopausal woman undergoing hysterectomy for endometriosis. These two cell lines were seeded in a 24-well-plate at a density of 1x10⁵ cells/well in Keratinocyte-Serum Free medium (Invitrogen) supplemented with 0.1 ng/ml human recombinant epidermal growth factor (EGF, Sigma-Aldrich), 0.05 mg/ml bovine pituitary extract (Invitrogen), and additional 44.1 mg/ml calcium chloride (final concentration 0.4 mM). Cells were infected with 10⁴ IU of FUGW resulting in multiplicity of infection (MOI) of 0.1, for 2 days at 37°C. Cells were washed twice with PBS and then genomic DNA (gDNA) was extracted using the blood DNA mini kit (Qiagen) and eluted in 30 µl elution buffer. For qPCR analysis, 100 ng of total gDNA was used in a reaction volume of 25 µl. Primary vaginal cells extracted from mouse vaginal lavage fluid were cultured in epithelial cell medium (ECM). ECM consists of equal volumes of phenol-red-free DME (Sigma-Aldrich) and Ham's F-12 medium (Invitrogen) supplemented with 10% FCS, 100 mg/ml streptomycin, 100 IU/ml penicillin, 1 mmol/ml L-glutamine, and 10 ng/ml EGF. The vaginal cells at a density of 5x10⁴ cells in 500 µl volume were infected with 10⁴ FUGW TU corresponding to a MOI of 0.2 for 1 day at 37°C. Cells were washed twice with PBS and then gDNA was extracted and eluted in 30 µl elution buffer. For PCR, 40 ng of total gDNA was used in a reaction volume of 25 µl.

### Mouse model and FUGW virus challenge

Six- to eight-week old female C57BL/6 mice purchased from Harlan (Zeist) were used throughout these studies. Animal studies were performed according to Swiss Animal Rights in the animal facilities of the Institute of Medical Virology, university of <Zurich, with permission by the Zurich-Vetrinary-Office (213/00). Mice were kept in conventional conditions with full access to food and water. All mice were treated s.c. with 2.5 mg of progestin (Depo-Provera, Pfizer) to synchronize the estrus cycle. One week later, the progestin-treated mice were anesthetized with isoflurane (ABBOTT AG) and challenged with an intravaginal inoculum of 20 µl 3% carboxymethyl cellulose sodium (CMC) medium (Sigma-Aldrich) containing 10⁴ IU FUGW with or without 25 µM ODNs in. Four hours later the mice were euthanized and vaginal lavage with 100 µl of sterile PBS was performed. Viral RNA was then extracted from these vaginal lavage fluids using the viral RNA mini kit (Qiagen).

### Detection of FUGW by Real-Time PCR

For qRT-PCR RNA was reverse transcribed into cDNA using the High Capacity cDNA Archive Kit (Applied Biosystems) according to the manufacturer's instructions. Primers and Probes flanking the PPT sequence of FUGW are: 5'-GAGGAGGTGGGTTTTCCAGT-3' (forward) (SEQ ID NO 10), 5'-GGGAGTGAATTAGCCCTTCC-3' (reverse) (SEQ ID NO 11), and - FAM-5'-ACCTTTAAGACCAATGACTTACAAGGCAGC-3'-TAMRA (probe) (SEQ ID NO 12); for mouse glyceraldehyde-3 phosphate dehydrogenase (mGAPDH), 5'-CTTCACCACCATGGAGAAGGC-'3 (forward) (SEQ ID NO 13), 5'-GGCATGGACTGTGGTCATGAG-'3 (reverse) (SEQ ID NO 14). : FAM-5'-CCTGGCCAAGGTCATCCATGACAACTTT-3'-TAMRA (SEQ ID NO 15); for human glyceraldehyde-3 phosphate dehydrogenase (hGAPDH), 5'-GTTCCAATATGATTCCACCC-3' (forward) (SEQ ID NO 16), 5'-GAAGATGGTGATGGGATTTC-'3 (reverse) (SEQ ID NO 17), FAM-5'-CAAGCTTCCCGTTCTCAGCC-TAMRA-3' (probe) (SEQ ID NO 18). All primers and probes were purchased from Microsynth. The cycling conditions were: 50°C for 2 min (1 cycle), 95°C for 10 min (1 cycle), 95°C for 15 sec and 60°C for 1 min (50 cycles). The results are presented in the figures as FUGW RNA or DNA corresponding to absolute copy numbers per assay.

### Statistical analysis

Viral RNA levels after treatment were compared using analysis of variance for repeated measures with Bonferroni post-hoc test applied to logarithmically transformed data. SPSS 13.0 (SPSS Inc. IL) was used for statistical analyses.
The statistical significance of the antiviral activity of ODN A and ODN AM in virions and in vitro was determined by student's T-test and the results were expressed as mean±SEM. (error bars in graph). These P-values were for two-tailed significance test. For the in vivo studies, viral RNA levels after treatment were compared by analysis of variance with Bonferroni post-hoc test applied to logarithmically transformed and PBS normalized data using SPSS 13.0 (SPSS Inc. IL). Differences were considered to be significant at P<0.05.

### Analysis of Humanized SCID Mice with HIV in vivo

For the evaluation of an antiviral effect of ODN A *in vivo*, we tested the inhibition of viral growth in a mouse model, with human peripheral blood lymphocyte grafted to severe combined immunodeficiency mice, the huPBL/SCID-mouse model (Balzarini et al, 1996, Mol Pharmacol, 50:394-401). We designed a regimen, which ensured a high ODN A concentration during the test period by continuous treatment. HIV and ODN A were applied together at day 15 after engrafting SCID mice with huPBL, followed by administration of ODN A every one to two days up to day 28, when HIV is usually detectable in HIV-infected huPBL-SCID mice .Three groups were used: HIV-infected mice treated with ODN A (n=6) or PBS (n=3) as negative control as well as non-infected, non-treated mice (n=4). No visible toxicity or unusual behavior or appearance was observed for any of the mice. At day 28 the mice were sacrificed and the p24 levels in plasma were determined by a p24 ELISA.

### Examples

### 1. Targeting the RNase H in virions

An ODN has been shown to have *in vivo* efficacy against a murine oncogenic retrovirus. The virus causes splenomegaly in mice and we were able to demonstrate reduction of disease progression. However, a murine oncogenic retrovirus does not reflect the situation of primary blood-borne HIV. We designed this ex vivo study to be as close to the human situation, it builds on the unique property that virus can be targeted before entering a cell. It therefore intervenes earlier than the inhibitors of reverse transcriptase, which only work inside of a cell.

The invention leads to suprising and unexpected data, in particular through clinical studies using plasma of HIV-1 infected patients and the antiviral potential of ODN. Here we demonstrate that HIV particles present in the plasma of infected individuals from Zurich and HIV field isolates from Africa including two drug-resistant strains can be treated before entering a cell with an ODN for activation of the viral RNase H and self-destruction of the virus. Plasma of 20 HIV-1-infected patients from Zurich and 10 HIV-1 isolates from Africa and drug-resistant strains were processed for ex vivo treatment. HIV-RNA of cell-free virions treated with ODN in the plasma was measured by quantitative reverse transcription polymerase chain reaction (qRT-PCR) (Fig. 2). The infectivity of the treated virions was tested on primary human peripheral blood mononuclear cells (pPBMCs).

ODNs were used to target the RNase H in HIV particles. The ODNs are designed to bind to the PPT of HIV-1 IIIB. As controls we used a scrambled ODN Sc or buffer PBS. The sequences of the PPT and ODNs are shown in Fig. 4a. ODN A-effects on HIV were analyzed by measuring HIV RNA levels by qRT-PCR. Primers were used that flank the PPT, designated as nef or UTR, close to the cleavage site to avoid other effects on the RNA. Since this is a highly variable region we used two primer pairs. The more conserved gag (gag) primers are similar to those used in routine diagnostics tests (Fig. 4b).

In order to verify the molecular mechanism of the treatment, HIV-PPT RNA together with the ODN A was allowed to form a local hybrid as substrate for the RNase H and cleavage of the RNA moiety. This is demonstrated with in vitro transcribed radioactively end-labeled RNA and virion-associated RT/RNase H from a sucrose gradient-purified patient-derived HIV preparation. The cleavage site of the viral RT/RNase H was verified with recombinant RT/RNase H and proven to correspond to the natural cleavage site, 5' of the ACU sequence (Fig. 4c).

In order to establish the conditions required for treatment of HIV in blood, we isolated plasma from the blood of healthy donors, spiked it with the cell culture-adapted strain HIV-1 IIIB, and treated it with ODNs for up to 24 hours in vitro. The amount of non-hydrolyzed RNA was determined and shown to be significantly reduced with time and dose (Fig. 4d-e).

### 2. Reduction of viral loads in plasma

20 HIV-1-containing blood samples of patients from Zurich with different histories were analyzed in this study immediately after isolation. The samples were supplied with information on plasma viral loads and CD4+ counts determined prior to this study (Fig 4f). We used HIV-containing plasma from patient 13 for which sufficient material was available to establish the conditions with two concentrations of ODNs and various times (8, 12 and 24h) (Fig. 5a). We then chose 8 h and 5 µM ODNs and tested HIV in the plasma of HIV-infected patients from Zurich. The amount of residual RNA was quantified by qRT-PCR with nef or UTR primers. The amount of non-hydrolyzed viral RNA was reduced more than 2-fold in 94 % and 31 % of the cases in comparison to PBS and ODN Sc, respectively (Fig. 5b and see below, Fig. 7).
The levels of initial viral RNAs of the patients varied. Several parameters contribute to the variability, such as the patients' histories, the sequences of the primer binding sites used for the PCR reaction, degree of the complementarity of the ODN A to the PPTs (see below) as well as components of the blood.

### 3. Inhibition of viral infectivity of plasma

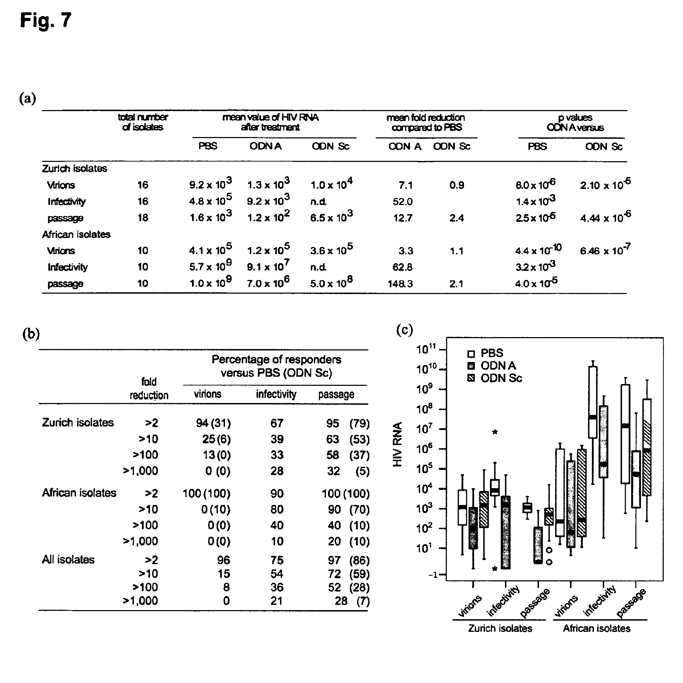

In order to reduce some of the variations, we tested the samples for infectivity as a second independent read-out, which correlates with viral RNA loads. To establish the conditions for infectivity of HIV-1-containing plasma, we treated one sample (patient 3) with ODN A for 4 h, infected normal human pPBMCs and cultured them for 7 days (Fig. 5c). The amount of HIV-1 RNA in the supernatant was reduced as determined by qRT-PCR using the conserved gag primers. The infectivity of all samples of the plasma from patients from Zurich was analyzed and showed a strong antiviral effect, ranging from two- to more than 1,000-fold (Fig. 5d and see below, Fig. 3 and Fig. 7).

In order to examine the antiviral effect of ODNs in the absence of blood factors and differences in viral titers, we used the supernatants from these infected cell cultures (first passage), matched the viral inputs to identical RNA copy numbers, treated them with ODNs and infected human pPBMCs. The supernatants (second passage) were analyzed after 7 days, showing even more pronounced the ODN A-mediated inhibition (Fig. 5e).

The PPT sequences targeted by the ODN A were determined by using RT-PCR-amplified products from selected patients (Fig. 5f). We found that single mutations in the 5'-region of the PPT and beyond were quite frequent, while the G-tracts were conserved, similar to sequences from the data base.

### 4. African and drug-resistant HIV isolates

We then analyzed well-characterized HIV-1 African primary field isolates and strains resistant against the protease inhibitor Saquinavir (Saqi) or the nucleoside analogue AZT (Azt) from the AIDS Reagent Program. These samples differed from Zurich isolates, since they were supplied after passaging in primary PBMCs. Therefore we present them as a separate group.

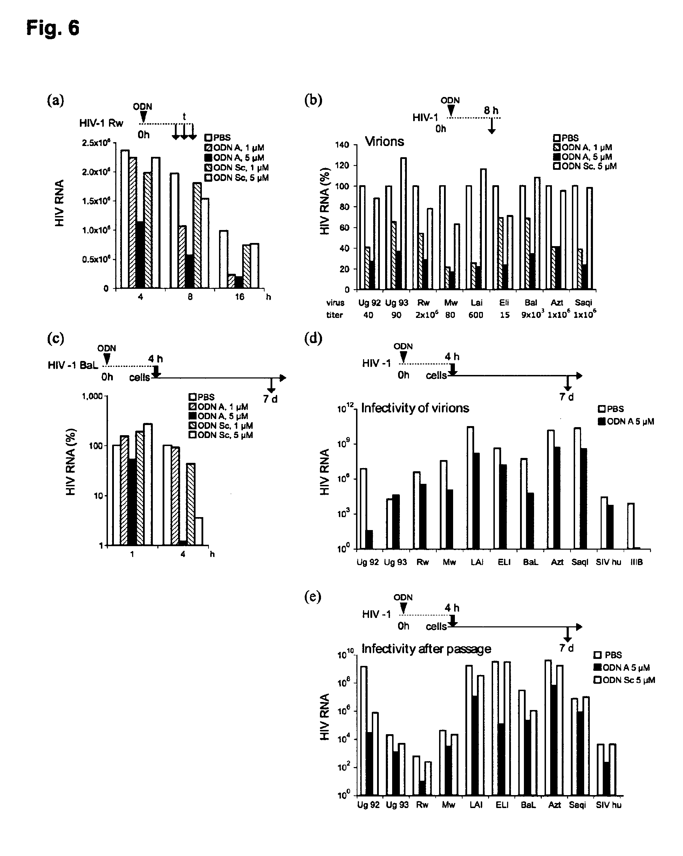

After demonstrating dose- and time-dependence with an HIV-1 isolate from Rwanda (Rw, Fig. 6a) we found that all strains were also susceptible to ODN A-mediated hydrolysis of viral RNA in virus particles (Fig. 6b) as well as ODN A-mediated inhibition of infectivity of the original starting material (Fig. 6 c-d) and after one passage in pPBMCs (Fig. 6e) with high statistical significance (see below, Fig. 7). Also for these strains mutations at the 5'-end of the PPT were determined (Fig. 5f) and showed similar variations.

The viral load in HIV-infected plasma samples from patients can be reduced by activation of viral RNase H by an ODN, demonstrating the effectiveness of the invention as an antiviral treatment. This leads to self-destruction of the virus particles. All ODN A-treated samples had a statistically significant decrease of the mean value of viral RNA compared to the control ODN Sc- and PBS-treated groups. Cell-free virions in plasma contained significantly less intact HIV-RNA upon treatment with ODN (p=0.000006), and their infectivity was decreased 52-fold (p=0.0014). In 39 % of the Zurich samples infectivity was reduced more than 10-fold, in 33 % more than 100-fold and in 28% more than 1000-fold (Fig. 7a-b). Also the isolates from Africa exhibited a 63-fold reduction in infectivity (p=0.0032) with 80 % of the isolates responding more than 10-fold, in 40 % more than 100-fold and in 10% more than 1000-fold (Fig.7a-b). The infectivity of treated virions was reduced on average 52-fold to 63-fold (Fig. 7a), 75% of all isolates showed a more than 2-fold reduction of infectivity, 54 % more than 10-fold, and 21 % more than 1,000-fold reduction (Fig. 7b). A combination of all experiments analyzed by Bonferroni post hoc test (Fig. 7c) revealed a significant decrease in viral load but also variability. All virus samples of patients with or without a history of antiviral therapy were susceptible to ODN A treatment. A second passage with standardized viral inputs strongly increased the specificity and confirmed the antiviral activity of ODN A to be statistically significant (Fig. 5e, 6e and 7b).

### 5. Analysis of ODNs against a lentiviral system

Human immunodeficiency virus (HIV) has been shown to undergo self-destruction upon treatment of cell-free virions with partially double-stranded oligodeoxynucleotides targeting the polypurine tract (PPT) of the viral RNA in the virus particle. The ODN forms a local hybrid with the PPT activating the viral RNase H to prematurely cleave the genomic RNA. Here the self-destruction of a recombinant lentivirus harboring the PPT of HIV in a mouse vagina model is described. It can be shown a decrease in viral RNA levels in cell-free virus particles and reduced reverse transcribed complementary DNA (cDNA) in virus- infected human and primary murine cells by incubation with ODNs. In the vagina simultaneous, prophylactic or therapeutic ODN-treatments led to a significant reduction in viral RNA levels.

We tested an application of ODN against a lentiviral vector applied to the mouse vagina as a model for sexual transmission. The lentiviral vector FUGW (Flap, ubiquitin promoter, GFP and WRE vector) contains the HIV-PPT sequence, which is essential for its single replication round. Here we demonstrate that FUGW present in the mouse vagina or in human vaginal or cervical cell lines can be treated with ODN A. We applied chemical modifications of the ODN such as phosphorothioates or 2'-O methyl groups to protect against nucleases and increase stability. In all cases, statistically significant reduction of FUGW virus RNA copies was observed with ODNs compared to their respective randomized sequence serving as negative controls. The approach we are using differs from previous ones, since it is based on the activation of a retroviral enzyme for destruction of the virus, instead of inhibition.

In order to establish an animal model for the antiviral activity of ODN we used the lentiviral vector FUGW (Fig 8A). FUGW is a lentiviral self-inactivating vector, which carries a green fluorescent protein, GFP, reporter driven by an internal ubiquitin promoter. Lentiviral particles are produced by co-transfection of the lentiviral vector plasmid cFUGW, the packaging plasmids pCMVΔR8.9 encoding gag and pol, and pHCMV-G encoding the Vesicular Stomatitis Virus envelope glycoprotein VSV-G. FUGW particles with the VSV-G envelope protein are infectious, but allow only one round of infection with no new virus production. These particles were used to infect cells or to conduct experiments in the mouse vagina (Fig. 8B). We targeted the PPT of FUGW with a hairpin-loop structured ODN, which hybridizes with one strand to the PPT and thereby presents a substrate for the RT/RNase H, which leads to cleavage of the RNA moiety in the local hybrid. The PPT of FUGW is identical to that of HIV as shown in Fig. 8C. The ODN contains phosphorothioates at the ends and in the central linker regions to protect against nucleases and to increase its stability and longevity. Additional modifications were 2'-O-methylated nucleotides, as were added to ODN AM. We designed control ODNs, which differed in the sequence of C and G, ODN S2 and ODN S2M (Fig. 8C).

To demonstrate ODN A-mediated hydrolysis by the RT/RNase H, we cleaved labelled PPT-RNA, which contains 167 nucleotides of the PPT region of FUGW. The PPT-RNA was hybridized to ODNs and incubated with RT/RNase H for 0.5 h at 37 °C. In parallel the PPT-RNA was sequenced by RNase T1 to determine the specific cleavage site. Only ODN A and ODN AM, but not ODN S2 or ODN S2M led to specific cleavage 5' to ACU, which is the natural cleavage site (Fig. 8D).

### 6. ODN A and ODN AM have an inhibitory effect on virions

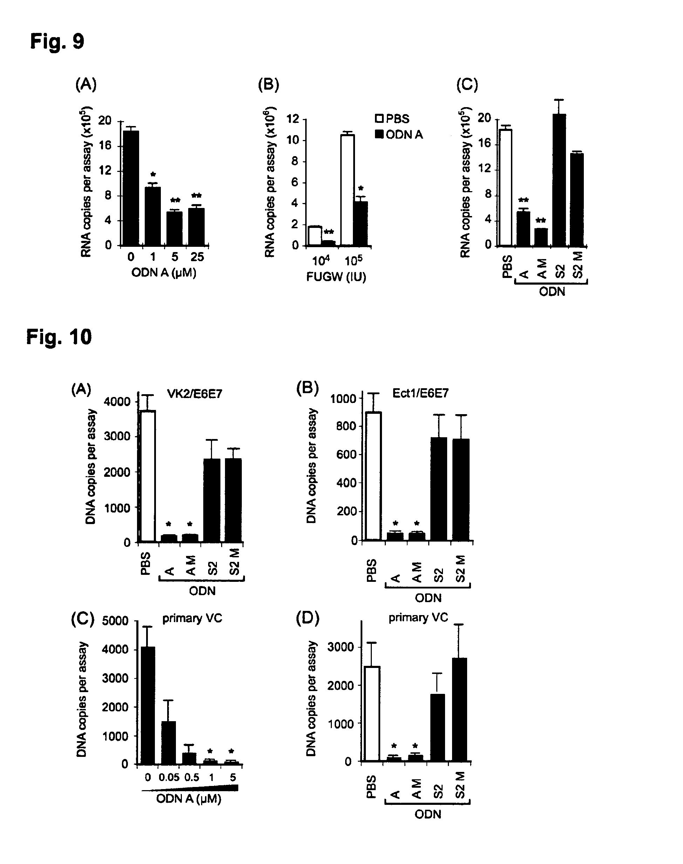

To test for cleavage of viral RNA in lentiviral particles, purified FUGW virions were treated with different concentrations of ODN A for 4 h at 37 °C. Then the viral RNA was purified and determined by qRT-PCR using primers flanking the PPT. These primers do not yield an amplification product, when the genomic RNA has been cleaved by RT/RNase H upon binding of ODN A to the PPT. The viral RNA levels in the virions were significantly reduced in a dose-dependent manner. With 5 µM ODN A the reduction was 71 % (p<0.01) and was used for further experiments (Fig. 9A). Similarly, when different virus titers were used, the reduction was less pronounced for higher virus titers (60 %, P=0.023, Fig. 9B) in comparison to lower titers (80 %, P=0.008). To assess the effect of all different ODNs on virions, cell-free FUGW particles were treated with ODN A, -AM, -S2, and -S2M for 4h at 37 °C (Fig. 9C). A reduction of FUGW RNA was observed for ODN A (71 %, P=0.0063 versus PBS, P=0.0337 versus ODN S2) and ODN AM (86 %, P=0.0037 versus PBS, P=0.0015 versus ODN S2M) but not for their respective controls, ODN S2 and ODN S2M. ODN AM was slightly more active than ODN A.

### 7. Effect of ODNs on FUGW-infected cells

FUGW transduces cells through one round of replication leading to provirus-containing cells, which then express GFP. However, no new virus progeny is produced from these cells, since FUGW is replication defective.
In order to analyze the effect of ODNs in human vaginal cells, we transduced the human vaginal (VK2/E6E7) and cervical (Ect1/E6E7) cell lines with FUGW. The cells were tested in the absence and presence of different ODNs. Genomic DNA (gDNA) was purified from the infected cells after 2 days incubation at 37 °C and analyzed by FUGW-specific qPCR (Fig. 10A and B) to measure the reverse transcribed cDNA or proviral DNA. A significant decrease of 95 % of FUGW DNA copies was determined for ODN A and ODN AM in comparison to the controls for both cell lines.

Next we used murine primary vaginal cells (VC), which were obtained by lavage of mouse vagina and infected them with FUGW using different concentrations of ODN A from 0.05 to 5 µM (Fig. 10C). The isolated DNA was analyzed by qPCR. The results indicate that ODN A exhibited a dose-dependent reduction of FUGW DNA copies with the strongest antiviral effect (P=0.0297) at 1 µM. Subsequently, all ODNs were tested under the same conditions (Fig. 10D). As can be seen, a significant decrease in FUGW DNA copies was observed for ODN A (96 %, P=0.02 versus PBS and P=0.0249 versus ODN S2) and ODN AM (94 %, P=0.0233 versus PBS and P=0.0406 versus ODN S2M) compared to their respective controls (Fig. 10D).

### 8. Assessment of ODNs in vivo

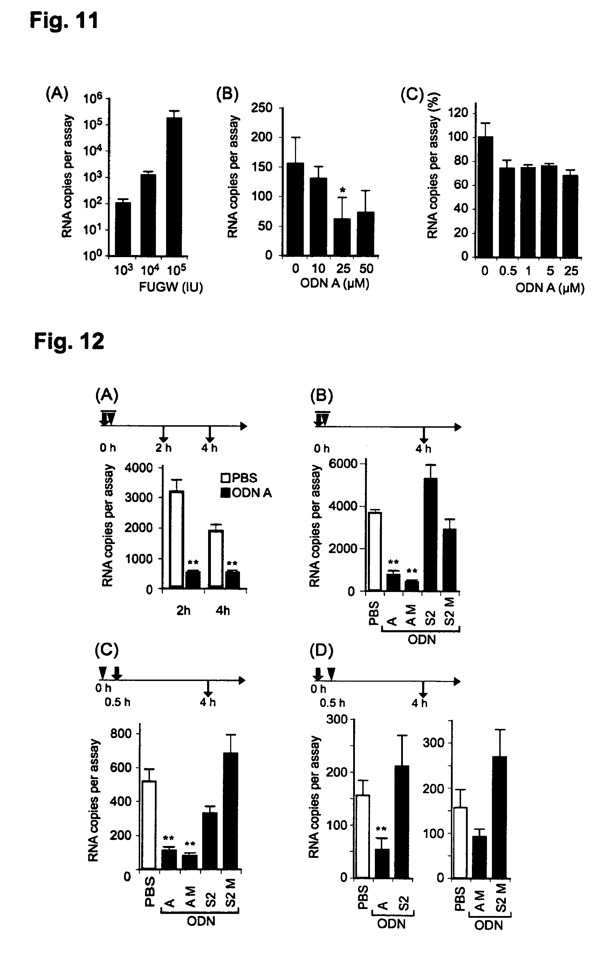

In order to establish the conditions for ODN treatment in the in vivo model, we applied three increasing doses of FUGW (10³, 10⁴ and 10⁵ IU) into the vagina in 20 µl containing 3 % carboxymethyl cellulose, in order to prevent leakage of the virus. After 4 h infection, a vaginal lavage was performed, viral RNA purified and genomic FUGW RNA detected by qRT-PCR using PPT-flanking primers. The lavage fluid contained FUGW RNA levels, which correlated with the dose of the applied lentivirus (Fig. 11A). Based on these results, we used 10⁴ TU FUGW for further experiments. First we assessed the effect of ODN A on FUGW in the mouse vagina by co-application of three concentrations of ODN A for 4 h. The reduction of FUGW cDNA was dose-dependent. At a concentration of 25 µM the reduction was 61 % compared to absence of ODN (P=0.0269 shown in Fig. 11 B).

In order to exclude the possibility that ODN A interfered with the RT-PCR system, we performed a control. We used purified FUGW RNA from 10⁴ TU and added increasing ODN A concentrations and subsequently purified the amount of RNA. It was then tested by RT-PCR and amplification was only weakly affected and this effect was independent on the amount of ODN A (Fig. 11 C).

### 9. Use and effect of ODNs in lentiviral mouse vaginal model

For the evaluation of ODN-mediated inhibition of FUGW in vivo we decided to apply three different treatment regimens, namely co-application, prophylactic and therapeutic regimens. First FUGW and 50 µM ODN A were co-applied and FUGW RNA levels were determined after 2 and 4 hours. A significant four- to sevenfold decrease (P<10⁻⁶) of viral RNA levels in the presence of ODN A was observed (Fig. 12A). For the following treatments, 10⁴ FUGW TU and 25 µM ODNs were used. The co-application into the vagina of FUGW and different ODNs for 4 h showed that ODN A and ODN AM exerted a strong reduction of viral RNA in comparison to the controls ODN S2 and ODN S2M (Fig. 12B). In the prophylactic treatment regimen ODNs were applied 30 minutes (min) before lentivirus infection (Fig. 12C). Also under these conditions ODN A and ODN AM, but not ODN S2 or ODN S2M led to significantly decreased viral titers. Finally, in the therapeutic treatment (Fig. 12D) the lentivirus was applied 30 min before ODNs. In the case of ODN A the intravaginal titer of FUGW RNA was reduced by 78 %. A reduction by ODN AM was also detected (Fig. 12D).

Here we are demonstrating the role of the RNase H in the antiviral effect of ODNs in the mouse vagina. We first demonstrated the cleavage of the PPT-RNA in presence of ODN A and ODN AM in vitro and mapped the cleavage site (Fig. 8D), which is adjacent to the ACU site and identical to the natural RNase H cleavage site for generation of the RNA primer for the second strand DNA synthesis. Using lentiviral particles harboring the HIV-1IIIB-type 3'-PPT (Fig. 8A, B and C), we demonstrate the antiviral effect by incubating these particles with ODN A or ODN AM in vitro (Fig. 9). Accordingly, also the infectivity of the treated virions was significantly reduced with ODN A and ODN AM in human vaginal or cervical cell lines as well as in mouse primary vaginal cells (Fig. 10). Next we established an in vivo model to test the reduction of lentiviral genomic RNA in the mouse vagina and excluded the possibility that ODN A would interfere with the PCR reaction (Fig. 11). ODN A is not toxic since the vitality of vaginal and cervical cell lines is not affected by ODN A as determined by trypan blue exclusion and cell proliferation assay.

In the lentiviral mouse vaginal model, we are evaluating different therapeutic regimens. We are treating mice first by applying FUGW and ODNs simultaneously (Fig.12A and B). Then we infected the mice in the vagina with FUGW and applied ODNs earlier (Fig. 12C) or later (Fig. 12D). In these different treatment regimens, simultaneous, prophylactic or therapeutic, ODN A and ODN AM led to reduction of lentiviral load as summarized in Table I. ODN A significantly induced reduction of FUGW in all settings. Also ODN AM led to a reduction. It was slightly more active in simultaneous and prophylactic ODN applications. In these simultaneous and prophylactic settings, based on the statistical analysis of the distribution of our data, we expect between 30% and 38% responders with more than 10-fold reduction of the RNA levels when treated with ODN A and ODN AM. Whereas in the therapeutic treatment with ODN A, we expect 17% responders with 10-fold reduction of RNA levels (Table 1). Thus, ODNs are able to induce self-destruction of the viruses by degrading the viral RNA in the mouse vagina. Double-stranded ODNs are more stable than single-stranded DNA and this stability is increased by phosphorothioate-modifications in ODNs and by additional 2'O-methyl groups in ODN AM. Other chemical modifications are feasible and may improve the stability of the ODN significantly. Recently locked nucleic acid (LNA) DNA has been shown to be stable for weeks.

**Table 1: Reduction of FUGW RNA levels upon treatment with ODNs in the mouse vagina**

| ODN | n | Mean RNA level (%) | Std. Error (%) | 95% Confidence Interval | | p-values in comparison to | | | | percentage of more than 10-fold reduced RNA levels |
|---|---|---|---|---|---|---|---|---|---|---|
| | | | | Lower Bound (%) | Upper Bound (%) | ODN AM | ODN S2 | ODN S2M | PBS | |
| *Coapplication* | | | | | | | | | | |
| ODN A | 87 | 30.36 | 0.09 | 21.64 | 42.59 | 1 | 5.12x10⁻¹⁴ | 6.96x10⁻⁵ | 7.49x10⁻¹³ | 33 |
| ODN AM | 30 | 18.95 | 0.10 | 11.87 | 30.32 | n.a. | 1.30x10⁻¹² | 1.37x10⁻⁵ | 1.78x10⁻¹⁰ | 38 |
| ODN S2 | 37 | 210.70 | 0.10 | 136.04 | 325.90 | | n.a. | 0.736 | 0.276 | 1 |
| ODN S2M | 20 | 110.58 | 0.10 | 64.34 | 190.03 | | | n.a. | 1 | 4 |
| PBS | 87 | 100.00 | 0.09 | 71.28 | 140.30 | | | | n.a. | 3 |
| | | | | | | | | | | |
| *Prophylactic* | | | | | | | | | | |
| ODN A | 40 | 20.88 | 0.12 | 15.11 | 28.86 | 1 | 0.006 | 1.36x10⁻⁵ | 1.22x10⁻⁸ | 30 |
| ODN AM | 15 | 14.65 | 0.13 | 8.72 | 24.63 | n.a. | 0.001 | 3.71x10⁻⁸ | 5.94x10⁻⁸ | 35 |
| ODN S2 | 30 | 53.69 | 0.12 | 37.17 | 77.43 | | n.a. | 0.336 | 0.128 | 0 |
| ODN S2M | 15 | 106.88 | 0.13 | 63.66 | 179.73 | | | n.a. | 1 | 2 |
| PBS | 40 | 100.00 | 0.12 | 72.35 | 138.21 | | | | n.a. | 1 |
| | | | | | | | | | | |
| *Therapeutic* | | | | | | | | | | |
| ODN A | 15 | 22.25 | 1.04 | 14.46 | 34.22 | 0.013 | 4.49x10⁻⁶ | 2.56x10⁻⁸ | 1.98x10⁻⁶ | 17 |
| ODN AM | 15 | 61.43 | 1.04 | 39.94 | 94.48 | n.a. | 0.340 | 0.012 | 0.696 | 2 |
| ODN S2 | 15 | 118.96 | 1.04 | 77.28 | 182.82 | | n.a. | 1 | 1 | 0 |
| ODN S2M | 15 | 171.55 | 1.04 | 111.48 | 263.74 | | | n.a. | 0.452 | 0 |
| PBS | 30 | 100.00 | 0.98 | 73.75 | 135.59 | | | | n.a. | 0 |

The data of all in vivo experiments in the mouse vagina are summarized and statistically analyzed in Table 1. The number of mice, the mean RNA level and standard error, the lower and upper bound of the 95% confidence interval as well as P-values for each combination of ODNs are shown. P < 0.05 are considered as significant. From the distribution of differences the percentage of responders with a more than 10-fold reduced FUGW RNA levels were calculated (n.a., not applicable).

The use of ODN to counteract HIV infection is a novel approach, which is based on the activation rather than the inhibition of a retroviral enzyme. The activation of the viral RNase H and thereby induced self-destruction of the RNA by treatment of virus particles through this approach demonstrates an antiviral therapeutic for the inactivation of viral infectivity outside of the cell. One of the most prominent examples where this is relevant would be prevention of sexual and mother-to-child transmission.

### 10. Antiviral activity of ODN A on HIV-infected human T-cells

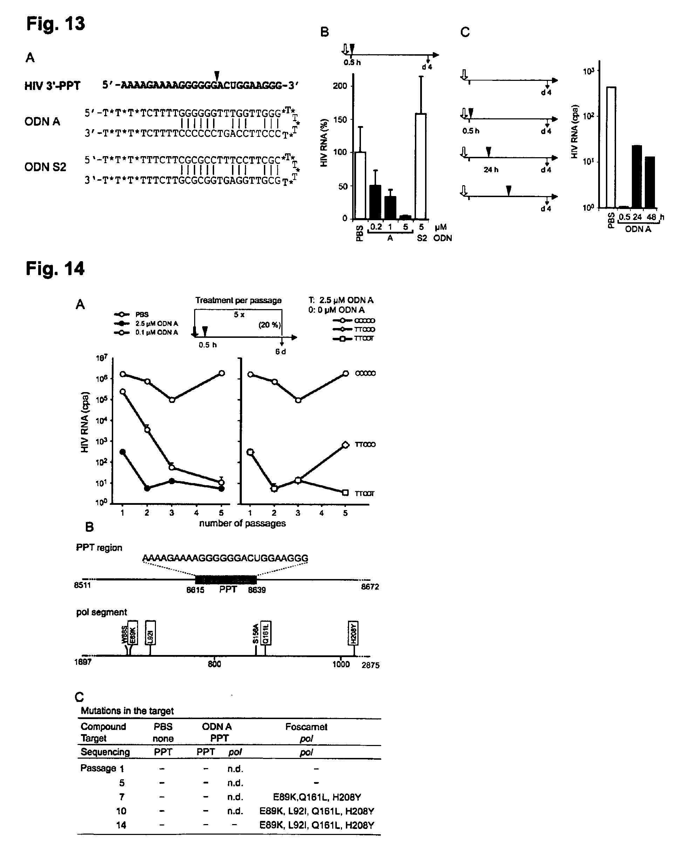

To further characterize the ODN we analyzed firstly induction of resistant mutants by ODN A and secondly its effect on HIV in a humanized mouse model. Rapidly emerging drug resistant mutants are a problem, yet we are targeting the highly conserved PPT which coevolved with the reverse transcriptase (RT) and hypothesized that it might be less prone to mutations. To analyze this we determined the dose-dependence of the antiviral activity of ODN A on the human T-cell line C81/66-45. The cells were infected with HIV-1 for 30 minutes, washed, treated with increasing concentrations of ODN A (0.2, 1, and 5 µM final concentration) or the scrambled control ODN S2 (5 µM), and viral growth was allowed to continue for 4 days at 37°C (Fig. 13A-B). Viral RNA was determined from the supernatant by gag-specific quantitative RT-PCR. ODN A inhibited viral growth with a half maximal inhibitory concentration (IC50) of approximately 20 nM, while 5 µM of the control did not. Next we tested different time points of treatment at a concentration of 5 µM ODN A (Fig. 13C). When ODN A was added 0.5 h post infection, the viral RNA level was reduced approximately 500-fold, whereas later addition of ODN A at 24 or 48 h led to a reduction of approximately 20 to 30-fold most likely since the first round of replication was not affected.

Then we tested the effect of a continuous treatment with ODN A to further reduce the viral load. We established conditions for serial passages to maintain HIV without treatment and then performed serial passages in the presence of 0.1 or 2.5 µM ODN A and PBS as control (Fig. 14A, left panel). We observed a rapid progressive decrease of the viral load for 2.5 µM ODN A close to the detection limit within 2 passages. The lower concentration was also effective, but caused less reduction of viral load and reached the detection limit only after 4 passages. The administration of ODN A could even be transiently omitted for one or two passages (Fig. 14A, right panel, indicated by T for one treatment, TT for two treatments and 0 for no treatment). After passage 2 with treatment (TT) virus was down but grew up in the absence of treatment (TT000). This was not due to resistance, because resumed treatment (TTTOOT) could revert the effect.

### 11. Induction of HIV mutation by ODN treatment

In order to test, whether ODN A treatment induced mutations we performed medium-term serial passages for three months in the presence of ODN A. We applied the same regimen as before for each passage, used 50 % of the supernatant at day 6 for the next passage, and applied in parallel to ODN A also an inhibitor to determine whether we could induce resistant HIV mutants and possibly escape mutations. The RT inhibitor Foscarnet known to induce specific mutations in its target *pol* gene of the RT was used as a positive control and PBS as negative control. Sixteen passages were performed. Following an initial drop in the viral load in the supernatants for ODN A and Foscarnet the titer reached a plateau after passage 10 (data not shown). Samples of passages 1, 5, 7, 10, and 14 were analyzed for nucleotide variations in the 3' PPT region and the *pol* gene. We did not detect any mutants in the 3' PPT or its flanking regions as well as in *pol* for ODN A and PBS treatment (Fig. 14B-C). In contrast upon treatment with Foscarnet specific mutations in *pol* appeared already at passage 7. Virus recovered from the ODN A-treated passages did not exhibit a higher resistance against ODN A as virus from first passages or PBS-treated passages, since the IC50 was comparable for both (data not shown) indicating absence of mutations affecting inhibition by ODN A. In conclusion these results suggest that under conditions causing mutations of HIV by Foscarnet, ODN A did not exhibit mutations in its direct targets, the PPT or the RT.

### 12. Inhibition of viral growth in the human peripheral blood lymphocyte / severe combined immune deficiency (huPBL/SCID)-mouse model

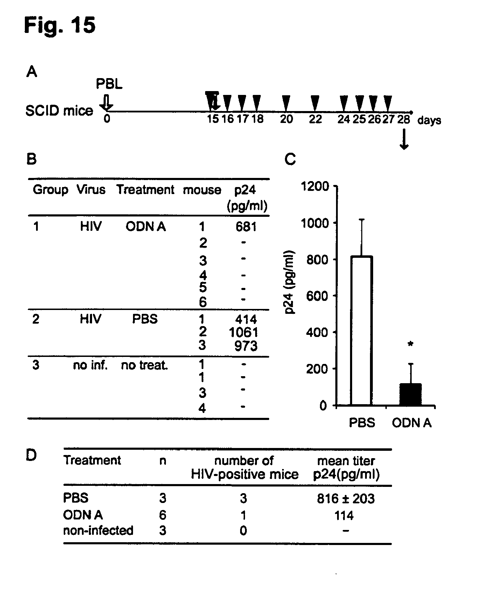

For the evaluation of an antiviral effect of ODN A *in vivo,* we tested the inhibition of viral growth in the huPBL/SCID-mouse model. Based on the results described above and published previously we designed a regimen, which ensured a high ODN A concentration during the test period by continuous treatment. HIV and ODN A were applied together at day 15 after engrafting SCID mice with huPBL, followed by administration of ODN A every one to two days up to day 28, when HIV is usually detectable in HIV-infected huPBL-SCID mice (Fig. 15A). Three groups were used: HIV-infected mice treated with ODN A (n=6) or PBS (n=3) as negative control as well as non-infected, non-treated mice (n=4). No visible toxicity or unusual behavior or appearance was observed for any of the mice. At day 28 the mice were sacrificed and the p24 levels in plasma were determined by a p24 ELISA (Fig. 15B, C). As expected all HIV-1-infected mice treated with PBS exhibited detectable levels of p24 corresponding to an infection rate of 100 %. Mice infected with HIV and treated with ODN A did not allow any virus detection in 5 out of 6 cases. Only in one out of 6 mice virus was detectable, corresponding to an apparent infection rate of 17 %, demonstrating that ODN A indeed reduced HIV titers in this in vivo humanized mouse model (Fig. 15D).

Mutagenesis of HIV during serial passage in combination with ODN and the effect of ODN against HIV in a humanized mouse model were analyzed. ODN A inhibits growth of HIV in vitro in a single passage by 100- to 1000-fold. When applied continuously a more than 10⁵-fold reduction is achieved within two passages at 2.5 µM, and in four passages at 100 nM, without using a transfection reagent. Interruption of ODN A treatment allowed virus to grow - but it remained sensitive and could be suppressed again (Fig.14). Passaging of the virus for 3 months did not induce mutations in its target sequence, the PPT, or in a region of the pol segment, which exhibited mutations upon treatment with Foscarnet already after 3 weeks.

This demonstrates an unexpected advantage over other HIV treatments such as Foscarnet. Using ODN mutagenesis of HIV does not occur as rapidly, and thus HIV remains sensitive to treatment. The in vivo efficacy of ODN A and sequence stability of the PPT are important prerequisites for further possible applications of ODN A. Since we are specifically inactivating the cell-free virus particles with ODNs, a mode of application could be Pre-Exposure Prophylaxis (PrEP) e.g. in form of a microbicide - against a major route of infection in the third world.

### FIGURES AND LEGENDS

- Fig. 1: Schematic: Mechanisms of "silencing of RNA"
- Fig. 2: Schematic: Ex vivo ODN treatment
- Fig. 3: Serial passage in the presence of ODN
- Fig. 4: In vitro mechanism of HIV treatment with ODN
- Fig. 5: Reduction of viral loads in plasma from Zurich patients
- Fig. 6: Reduction of viral loads in plasma from African patients
- Fig. 7: Effect of ODNs on infectivity of HIV particles
- Fig. 8: Analysis of ODNs and lentiviral system
- Fig. 9: ODN effect on recombinant lentiviral particles (FUGW)

- Fig. 10: ODN effect on FUGW infected cells
- Fig. 11: Assessment of ODN activity in vivo
- Fig. 12: Use and effect of ODNs in lentiviral mouse vaginal model
- Fig. 13: ODN A inhibits HIV infection of T-cells in vitro
- Fig. 14: Serial passages of HIV-1 under ODN A treatment
- Fig. 15: Inhibition of HIV-1 in vivo

### DETAILED DESCRIPTION OF THE FIGURES

Fig. 1. Schematic of the different "silencing of RNA" mechanisms.

Fig. 2. Serum isolated from HIV infected individuals, was treated and the effect of a short DNA on viral infectivity after treatment ex vivo was characterized.

Fig. 3. Infectivity of the virus in the plasma was characterized by wild-type polymerase chain reaction as well as infectivity of the treated virus on primary peripheral blood mono-nucleocyte cells (PBMC). 20 patients from Zurich and 10 field isolates from Africa were treated. Also African HIV strands have been analyzed including multi drug resistant cases. The infectivity was thousand-fold reduced in 18-37 % of the cases.

Fig. 4. (A) Sequences of extended PPT-region of HIV-1 and ODNs. The cleavage site by RT/RNase H is indicated by an arrowhead. (B) Detection of HIV-1 by qRT-PCR. The primers (one-sided arrows) and probes (lines with closed circles), and coordinates on the HIV-1 reference sequence (Gene Bank accession number 9629357) as well as the PPT (black box) and the RT/RNase H cleavage site are shown schematically. The primer pairs for detection were designated as gag, nef and UTR. (C) Cleavage of HIV-PPT RNA by RT/RNase H. HIV PPT-RNA annealed to oligonucleotide was incubated with purified virions of patient 8898, permeabilized with 0.1 % NP-40, for the indicated time at 37°C or without (-) or with (+) recombinant RT/RNase H for 30 minutes. In parallel HIV-PPT-RNA, partially digested with RNase T1, was used as a marker. (D) Time dependence. HIV-1 IIIB supplemented with human healthy serum was treated with 1 µM ODN A, ODN Sc or PBS (arrow head on dotted line) for the indicated times at 37°C. The viral RNA was extracted (arrows) and quantified by qRT-PCR. The mean values of three independent experiments are shown. (E) Dose dependence. HIV-1 IIIB virions were incubated with the indicated concentrations of ODN A for 8h at 37°C and the amount of RNA measured by qRT-PCR. (F) (Left) Characteristics of patients from Zurich infected with HIV-1. HIV-1 plasma loads (copies/ml) measured by COBAS Taqman assay (Roche, Switzerland), CD4+ cell-count (cells/µl) and anti-retroviral therapy status of the patients was monitored prior to enrollment. (Right) African isolates were obtained from the ARRRP (n.a., not available).

Fig. 5. Analysis of ODN A-mediated effects on HIV-1 isolates from patients from Zurich. (A) Time and dose dependence. HIV-1- containing plasma of patient 13 was treated with ODNs at 37°C as indicated and the levels ofviral RNA were measured by qRT-PCR. ODN Sc or PBS served as controls. (B) Determination of viral RNA in virions in plasma. HIV-1 containing plasma of patients 1 to 19 was treated with 5 µM ODNs at 37°C for 8h at 37°C and the levels of viral RNA were quantified. (*, not done). Patient 20 failed in some assays and was not pursued further. (C) Inhibition of infectivity. HIV-containing plasma of patient 3 was incubated with two concentrations of ODNs as indicated at 37°C. The plasma was then used for infection (thick arrow) of primary human healthy pPBMCs. After 7 days (7d) viral RNA was extracted from the supernatant of infected cells and the levels were measured. (D) Infectivity of virions in plasma. Plasma from patients 1 to 19 was treated with ODN A for 4 h and then used for infection of pPBMCs as described in (C). HIV RNA levels were determined after 7d. (E) Infectivity after passage. The supernatants of infected cells recovered after 7 d shown in (D) were adjusted to 2 x 10⁵ copies/ml, treated with ODNs for 4 h, and tested for infectivity on pPBMCs as described in (C). (F) Sequences of the PPTs of HIV from selected Zurich patients and from African isolates were derived from PCR products generated using the supernatants of (e) after two passages. The HIV-1IIIB extended PPT is indicated with the RT/RNase H cleavage site (arrow head) with differences to the PPT underlined.

Fig. 6. Analysis of ODN A-mediated effects on African HIV field isolates. (A) Time and dose dependence. An HIV-1 isolate from Rwanda (Rw) was treated with ODNs at 37°C as indicated and the levels of viral RNA were measured by qRT-PCR. ODN Sc or PBS served as controls. (B) Determination of viral RNA in virions. HIV-1 strains were treated with 5 µM ODNs for 8h at 37°C and the levels of viral RNA were measured. (C) Optimization of ODN A-mediated inhibition of infectivity. HIV-1 BaL was incubated with ODNs for 1 and 4 h at 37°C and used for infection of pPBMCs. Viral RNA levels were determined in the supernatants of the infected cells after 7 d and quantified. (D) Inhibition of infectivity of virions. The indicated strains were treated with ODN A for 4 h and then used for infection. RNA levels were quantified in the supernatants after 7d. (E) Infectivity after passage. The supernatants described in (d) were adjusted to 2 x 10⁵ copies/ml, treated with ODNs applied to pPBMCs and tested after 7d for viral RNA levels.

Fig. 7. Analysis of the response of HIV-1 isolates to ODN A treatment of virions, infectivity of treated primary and passaged virions (A) Changes in viral load. Total number of patients, mean values of HIV-RNA in number of copies per assay after treatment of Zurich and African HIV isolates, mean fold reduction and p-values comparing ODN A-treated with PBS- or ODN Sc-treated groups are shown (n.d., not done). (B) Degree of response. The percentage of responders was grouped based on the fold reduction for each group of HIV isolates. (C) Box-plot presentation of the data used for Bonferroni Post-hoc analysis. Asterisks and circles represent outliers.

Fig. 8. Oligodeoxynucleotides and FUGW system and RT/RNase H. (A) Recombinant lentiviral vector cFUGW harboring the HIV-3'-PPT sequence. The vector contains the cytomegalovirus enhancer (CMV), the HIV-1 flap region, the human ubiquitin C promoter for transcription of GFP, the woodchuck hepatitis virus posttranscriptional regulatory element (WRE) and an inactive 3'-ΔLTR. (B) Schematic representation of the three-plasmid expression system used for generating lentiviral particles by transient transfection consisting of cFUGW (lentiviral plasmid), pHCMV-G (VSV-G), and pCMVΔR8.9 (gag-pol). Viral particles produced were used either for transduction of cells or for in vivo application. (C) Sequences of the FUGW-3'-PPT and of ODNs. The cleavage site by RT/RNase H is indicated by an arrowhead (phosphorothioates, *; 2'-O-methyl, m) (D) Cleavage of PPT-RNA by RT/RNase H. PPT-RNA annealed to ODNs was incubated with recombinant RT/RNase H and subjected to denaturing PAGE. In parallel PPT-RNA partially digested with RNase T1, was used as a marker.

Fig. 9. Effect of ODNs on FUGW virions. (A) Cell-free FUGW virions were incubated either with ODN A at the indicated concentrations for 4h at 37°C. The genomic RNA of lentiviral particles was purified and quantified by PPT-specific qRT-PCR. Bars represent mean values ± SEM. *, P<0.05; **P<0.01. n=3. (B) Titration of FUGW-virions (10⁴ or 10⁵ IU) with 5µM ODNs for 4h at 37°C. RNA was quantified by qRT-PCR. Bars represent mean values ± SEM. *, P<0.05; **P<0.01. n=2. (C) Effect of 5 µM ODNs on 10⁴ FUGW TU for 4h at 37°C. RNA was quantified by qRT-PCR. Bars represent mean values ± SEM. **P<0.01. n=3.

Fig. 10. Effect of ODNs on FUGW-infected cells. (A-D) Cells were infected with 10⁴ FUGW TU in the presence of 1 µM ODN as indicated and incubated for 1 or 2 d. DNA was isolated and quantified by qPCR using human or mouse GAPDH for standardization. Data are shown as mean ± SEM. *, P<0.05. VK2/E6E7 (B, n = 6) and Ect1/E6E7 (C, n = 6). Primary vaginal cells extracted from vaginal lavage fluid were infected in the presence of the indicated concentrations of ODN A (E, n = 3) or different ODNs (D, n = 6) for 1 d.

Fig. 11. Establishing the lentivirus mouse vagina model. (A) Different amounts of FUGW were applied in the mouse vagina. After 4 h a vaginal lavage was performed and the RNA isolated from the lavage fluid and analyzed by PPT-specific qRT-PCR. Bars represent mean values ± SEM of RNA. n =7 mice per group. (B) Titration of ODN A intravaginally with 10⁴ FUGW TU. FUGW was applied together with increasing concentrations of ODN A for 4 h and FUGW RNA was measured as described in (A). Bars represent mean values ± SEM. *, P<0.05. n = 4 mice per group. (C) ODN does not interfere with RT-PCR assays. Purified FUGW RNA was incubated for 1 h with different concentrations of ODN A, repurified and RNA levels determined by qRT-PCR performed. Bars represent mean values ± SEM. n = 3.

Fig. 12. Effect of ODN treatment on FUGW in the mouse vagina. C57BL/6 mice were treated with 10⁴ FUGW TU and ODN in the vagina. Two or four hours later RNA was extracted from vaginal lavage fluids and FUGW RNA levels were determined by qRT-PCR. Thick arrow, FUGW infection; arrowhead, treatment with ODN; thin arrow, vaginal lavage. Bars indicate mean values ± SEM of RNA. **, P<0.01. (A, B) Co-application of FUGW and 50 µM ODN A (A, n= 50 mice per group) and with different ODNs at 25 µM (B, n = 16 mice per group). (C) For the prophylactic regimen 25 µM ODNs were applied 0.5 h before FUGW. n=13 mice per group. (D) In a therapeutic setting FUGW was applied 0.5 h before treatment with 25 µM ODNs. n=13 mice per group.

Fig. 13. ODN A inhibits HIV in vitro. (A) Sequences of the HIV-1 3'-PPT and ODNs used. The cleavage site of RNase H is indicated by an arrowhead and phosphothioates by asterisks. (B) Dose-dependent inhibition of HIV by ODN A. C81-66/45 cells (1 x 10⁵/ml) were infected with HIV-1 (open arrow, multiplicity of infection = 0.02) for 30 min. at 37°C, then washed (vertical line) and seeded in 48-well dishes prefilled with medium and ODN A (closed arrowhead) to achieve the indicated final concentration. At d4 supernatants were harvested and viral titers determined by nefspecific qRT-PCR (vertical arrow). Bars represent the mean of relative HIV RNA levels in % +SE of a representative experiment performed in triplicates. Bars represent the mean of HIV RNA levels in copies per assay (cpa). (C) Treatment at different time points was performed as indicated in the scheme. At later time points ODN A was directly added to the infected cells. Bars represent the mean of HIV RNA levels in copies per assay (cpa).

Fig. 14. Serial passages of HIV-1 under ODN A treatment. (A) Short-term serial passage. Infections, ODN A treatment and analysis was performed in triplicates as described in Fig. 1B without washing after infection. 20 % of the harvested supernatant was used for the next round of infection. The graph represents the viral titer in supernatants of the indicated passage for continuous (left panel) or discontinuous treatment (right panel) as indicated. The character strings indicate the history of individual series consisting of passages with (T) and without (O) treatment. The position in the string refers to the number of the passage. (B) Medium-term serial passages and analysis of mutants. Serial passages were performed for three months comprising 16 passages with few modifications as described in A. 50 % of the supernatant was used for the next passage, and a second spike of ODN A was applied 4 h after infection. ODN A was applied at 5 µM and Foscarnet at 100 µM. Viruses from passages 1, 5, 7, 10 and 14 were analyzed by population sequencing for mutations in the target regions. The scheme indicates the sequenced 3'-PPT (8615-8639) region (8511 to 8672) and *pol* segment (1697 to 2875). Coordinates refer to the genomic HIV-1 NCBI reference sequence NC_001802.1. Known mutations characteristic for resistance against Foscarnet are indicated by single letter amino acid code followed by position and the amino acid replacement. Mutations found in this study are boxed. (C) Summary of mutations. The table summarizes treatment, target, sequenced regions and found mutations for the indicated passages.^{∼}, no mutation detected, n.d., not done.

Fig 15. Inhibition of HIV-1 in vivo. (A) SCID mice were injected with PBL at day 0 (open arrow) and colonization of the mice was allowed for two weeks. At day 15 HIV-1 (thick arrow) and ODN A (filled triangle) were mixed and injected together (overline). ODN A was injected i.p. every one or two days as indicated (filled triangles). At day 28 blood samples were taken, plasma was prepared and p24 levels were measured. (B) The three groups tested were HIV-infected mice treated with ODN A (n=6) or PBS (n=3) as control, and non-infected, non-treated mice (n=4). The p24 levels are shown ^{~}(, not detected). (C) The graph indicates the mean p24 levels ± SE for PBS- and ODN A-treated HIV-1 infected mice. *p<0.05. D. The analysis of the response indicates the total number of mice (n), the number of HIV-1 positive mice, and the mean p24 levels ± SE.

## Claims

1. siDNA oligonucleotides, capable of binding to one or more RNA target regions of Human Immunodeficiency virus (HIV), as an antiviral therapeutic for the treatment and inactivation of cell free virus particles before infection and / or for the treatment and prevention of HIV infections inside the cell, whereby the siDNA oligonucleotides are consisting of an antisense-strand homologous to the viral RNA target and a second strand, partially complementary to the antisense-strand.

2. siDNA oligonucleotides, capable of binding to one or more RNA target regions of Human Immunodeficiency virus (HIV), as an antiviral therapeutic for the treatment and inactivation of cell free virus particles before infection and / or for the treatment and prevention of HIV infections inside the cell, whereby the siDNA oligonucleotides are consisting of an antisense-strand fully or almost fully homologous to the viral RNA target and a second strand, partially complementary to the antisense-strand forming a partially double stranded hairpin-loop-structured oligonucleotide, comprising G-clusters consisting of at least two G nucleotides in succession to allow tetrade or tetramer formation or tetra-helices or higher-ordered structures within the same siDNA oligonucleotide molecule (*cis* conformation) or through interaction with another siDNA oligonucleotide molecule (*trans* conformation).

3. siDNA oligonucleotides according to claim 1 or 2, whereby the siDNA molecule corresponds to one or more conserved HIV target regions, of at least 20 nucleotides in length, whereby the antisense strand of siDNA is more than 80%, more preferably more than 90% homologous to the target HIV viral RNA.

4. siDNA oligonucleotides according claim 1 or 2, whereby the second strand is connected to the antisense strand through a thymidine linker, preferred 4 nucleotides in length, whereby the second strand has a homology of 40 to 60% to the antisense-strand and is partially complementary within the hairpin-loop to the antisense-strand, and may be able to form triple helices by non-Watson-Crick base pairing with the viral RNA target.

5. siDNA oligonucleotides according to claim 1, wherein said siDNA comprise G-clusters consisting of at least two G nucleotides in succession to allow tetrade or tetramer formation or tetra-helices or higher-ordered structures within the same siDNA oligonucleotide molecule (*cis* conformation) or through interaction with another siDNA oligonucleotide molecule (*trans* conformation).

6. siDNA oligonucleotides according to claim 2 or 5, wherein at least two of the G-clusters are separated from each other by other nucleotides like A, T, C or G.

7. siDNA oligonucleotides according to one or more of the preceding claims, wherein the siDNA is stabilized by base modifications.

8. siDNA oligonucleotide according to one or more of the preceding claims, comprising the sequence SEQ ID NO 1 or 21.

9. Use of siDNA, or a combination of two or three siDNAs according to one or more of the preceding claims, as antiviral therapeutic for the treatment and inactivation of cell free virus particles before infection and / or for the treatment and prevention of HIV infections inside the cell, capable of binding to RNA target regions of HIV or different HIV virus variants, whereby different siDNA oligonucleotides may be combined in a cocktail as pharmaceutical agent.

10. Use according to claim 9, wherein the siDNA-containing pharmaceutical agent can also contain ribonucleotides, siDNA/RNA chimeras, or can be combined with siRNAs, or a cocktail of different siDNA oligonucleotides that target different HIV virus variants.

11. Use according to claim 9 or 10, wherein the siDNA will be applied to an infected cell or an infected individual with a transducing agent.

12. Use according to claim 11, whereby the transducing agent is selected from the following group: the virus itself, a replicating HIV virus particle which carries the siDNA into the cell during the process of infection, a liposome, transmembrane carriers or peptides.

13. Pharmaceutical composition as antiviral therapeutic for the treatment and inactivation of cell free virus particles before infection and / or for the treatment and prevention of HIV infections inside the cell comprising at least siDNA oligonucleotides according to one or more of the preceding claims, wherein said siDNA oligonucleotides are capable of binding to RNA target regions of HIV virus.

14. Pharmaceutical composition according to claim 13, comprising at least one siDNA oligonucleotide of the sequence SEQ ID NO 1 or 21.

15. Pharmaceutical composition according to claim 13 or 14, for the prevention of HIV infection during sexual transmission, during mother to child transmission, through Pre-Exposure Prophylaxis (PrEP), through the reduction of viral loads in body fluids, or for the treatment of multidrug-resistant patients.


SEQUENCE LISTING

<110>  Moelling, Karin

<120>  Method and compound for antiviral (HIV) therapy

<130>  545-09

<150>  EP 08075554.9
<151>  2008-06-13

<160>  21

<170>  PatentIn version 3.3

<210>  1
<211>  54
<212>  DNA
<213>  Artificial

<220>
<223>  synthetic oligonucleotide

<400>  1
ttttcttttg gggggtttgg ttgggttttc ccttccagtc cccccttttc tttt          54


<210>  2
<211>  24
<212>  DNA
<213>  Artificial

<220>
<223>  synthetic oligonucleotide

<400>  2
gcagccatgc aaatgttaaa agag                                          24


<210>  3
<211>  22
<212>  DNA
<213>  Artificial

<220>
<223>  synthetic oligonucleotide

<400>  3
tccccttggt tctctcatct gg                                            22


<210>  4
<211>  27
<212>  DNA
<213>  Artificial

<220>
<223>  synthetic oligonucleotide

<400>  4
tctatcccat tctgcagctt cctcatt                                       27


<210>  5
<211>  25
<212>  DNA
<213>  Artificial

<220>
<223>  synthetic oligonucleotide

<400>  5
caatgactta caaggcagct gtaga                                          25


<210>  6
<211>  23
<212>  DNA
<213>  Artificial

<220>
<223>  synthetic oligonucleotide

<400>  6
ttagcagaac tacacaccag ggc                                            23


<210>  7
<211>  32
<212>  DNA
<213>  Artificial

<220>
<223>  synthetic oligonucleotide

<400>  7
ttcactccca aagaagacaa gatatccttg at                                  32


<210>  8
<211>  26
<212>  DNA
<213>  Artificial

<220>
<223>  synthetic oligonucleotide

<400>  8
cctcaggtac ctttaagacc aatgac                                         26


<210>  9
<211>  19
<212>  DNA
<213>  Artificial

<220>
<223>  synthetic oligonucleotide

<400>  9
cccctggccc tggtgtgta                                                 19


<210>  10
<211>  20
<212>  DNA
<213>  Artificial

<220>
<223>  synthetic oligonucleotide

<400>  10
gaggaggtgg gttttccagt                                                20


<210>  11

<211>  20
<212>  DNA
<213>  Artificial

<220>
<223>  synthetic oligonucleotide

<400>  11
gggagtgaat tagcccttcc                                                    20


<210>  12
<211>  30
<212>  DNA
<213>  Artificial

<220>
<223>  synthetic oligonucleotide

<400>  12
acctttaaga ccaatgactt acaaggcagc                                         30


<210>  13
<211>  21
<212>  DNA
<213>  Artificial

<220>
<223>  synthetic oligonucleotide

<400>  13
cttcaccacc atggagaagg c                                                  21


<210>  14
<211>  21
<212>  DNA
<213>  Artificial

<220>
<223>  synthetic oligonucleotide

<400>  14
ggcatggact gtggtcatga g                                                  21


<210>  15
<211>  28
<212>  DNA
<213>  Artificial

<220>
<223>  synthetic oligonucleotide

<400>  15
cctggccaag gtcatccatg acaacttt                                           28


<210>  16
<211>  20
<212>  DNA
<213>  Artificial

<220>
<223>  synthetic oligonucleotide

<400>  16

gttccaatat gattccaccc                                                    20

<210>  17
<211>  20
<212>  DNA
<213>  Artificial

<220>
<223>  synthetic oligonucleotide

<400>  17
gaagatggtg atgggatttc                                                    20

<210>  18
<211>  20
<212>  DNA
<213>  Artificial

<220>
<223>  synthetic oligonucleotide

<400>  18
caagcttccc gttctcagcc                                                    20

<210>  19
<211>  54
<212>  DNA
<213>  Artificial

<220>
<223>  synthetic oligonucleotide

<400>  19
tttggggggt tcttcctcct ttcctttttc gcccgtccgt tgcgttgatt tttt               54

<210>  20
<211>  54
<212>  DNA
<213>  Artificial

<220>
<223>  synthetic oligonucleotide

<400>  20
ttttttcttc gcgcctttcc ttcgcttttg cgttggagtg gcgcgttctt tttt              54

<210>  21
<211>  54
<212>  DNA
<213>  Artificial

<220>
<223>  synthetic oligonucleotide

<220>
<221>  modified_base
<222>  (1)..(4)
<223>  2'-O-methylated uridine

<220>
<221>  modified_base
<222>  (51)..(54)

<223>  2'-O-methylated uridine

<400>  21
uuuucttttg gggggtttgg ttgggttttc ccttccagtc cccccttttc uuuu          54